(19) **Europäisches Patentamt European Patent Office Office européen des brevets**

(11) **EP 3 977 478 B1**

(12) **EUROPEAN PATENT SPECIFICATION**

(45) Date of publication and mention
of the grant of the patent:
**19.02.2025 Bulletin 2025/08**

(21) Application number: **20734465.6**

(22) Date of filing: **26.05.2020**

(51) International Patent Classification (IPC):
***G16H 50/20*** (2018.01)   ***G16H 50/70*** (2018.01)

(52) Cooperative Patent Classification (CPC):
**G16H 50/20; A61B 5/316; A61B 5/369;**
**A61B 5/374; A61B 5/4094; A61B 5/7264;**
A61B 5/291; A61B 5/4836; A61B 5/7257

(86) International application number:
**PCT/EP2020/064532**

(87) International publication number:
**WO 2020/239742 (03.12.2020 Gazette 2020/49)**

(54) **A COMPUTER IMPLEMENTED METHOD AND COMPUTER PROGRAM PRODUCTS FOR IDENTIFYING TIME-FREQUENCY FEATURES OF PHYSIOLOGICAL EVENTS**

COMPUTERIMPLEMENTIERTES VERFAHREN UND COMPUTERPROGRAMMPRODUKTE ZUR IDENTIFIKATION VON ZEITFREQUENZMERKMALEN VON PHYSIOLOGISCHEN EREIGNISSEN

PROCÉDÉ MIS EN ŒUVRE PAR ORDINATEUR ET PRODUITS DE PROGRAMME INFORMATIQUE PERMETTANT D'IDENTIFIER DES CARACTÉRISTIQUES DE FRÉQUENCE TEMPORELLE D'ÉVÉNEMENTS PHYSIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.05.2019 EP 19382423**

(43) Date of publication of application:
**06.04.2022 Bulletin 2022/14**

(73) Proprietor: **Universitat Pompeu Fabra**
**08002 Barcelona (ES)**

(72) Inventors:
• **VILA VIDAL, Manel**
**08005 Barcelona (ES)**
• **TAUSTE CAMPO, Adrià**
**08750 Molins de Rei (ES)**

(74) Representative: **Segui Quetglas, Margalida et al**
**Torner, Juncosa i Associats, S.L.**
**C/Pau Claris, 108, 1r 1a**
**08009 Barcelona (ES)**

(56) References cited:
**WO-A1-2016/200952     US-A1- 2007 250 133**
**US-A1- 2012 245 481**

• **VILA-VIDAL MANEL ET AL: "Detection of recurrent activation patterns across focal seizures: Application to seizure onset zone identification", CLINICAL NEUROPHYSIOLOGY, ELSEVIER, AMSTERDAM, NL, vol. 128, no. 6, 3 April 2017 (2017-04-03), pages 977 - 985, XP085018111, ISSN: 1388-2457, DOI: 10.1016/ J.CLINPH.2017.03.040**

**Description**

TECHNICAL FIELD

The present invention relates to a computer implemented method and to computer program products for identifying the characteristic time-frequency features of physiological events. In particular the invention can be used to extract the spectral features of the electrophysiological seizure onset patterns and to predict the epileptic focus.

BACKGROUND OF THE INVENTION

Recent technological advances in brain recording modalities have enormously increased the amount of available brain data sampled at various spatial and temporal scales. This opens up the possibility to develop algorithmic methods that read these data and extract relevant information for both scientific research and clinical practice. In this context, a variety of clinical and basic research problems that are associated to a specific temporal event where brain activity is either externally (e.g., electrical stimulation, drug administration) or internally (e.g., epileptic seizures) perturbed, can be analyzed in similar terms by a single methodology that uncovers patterns of physiological signals to determine its spatial, temporal and frequency extent.

Specifically, in a brain disorder such as epilepsy, there is a third of all patients that are drug-resistant, for which the success of the resective surgery critically depends on the accurate spatial definition of the epileptogenic zone (EZ). In particularly challenging cases, the use of invasive recording techniques to monitor intracranial electroencephalography (iEEG) activity might be required during the pre-surgical evaluation to determine the cortical areas to be resected.

Over the last decades, an increasing effort has been undertaken to develop quantitative tools for iEEG analysis to better characterize and understand how epileptic seizures are generated and propagated, a complex problem that involves both temporal and spatial variables. On the temporal domain, for instance, several studies have aimed to design methods that can efficiently and automatically detect interictal spikes high-frequency oscillations, or that can even predict seizures. In contrast, the development of automated methods to delineate the spatial localization of the seizure-onset zone (SOZ) remains challenging due to a number of reasons. The complex localization of the SOZ, the variable number and typology of seizures during the monitoring period and the variety of electrophysiological seizure-onset patterns that may occur even within one patient represent serious challenges to design a detection algorithm that is universally valid for all patients.

While the gold standard in SOZ identification is still retrospective visual inspection of iEEG recordings, several biomarkers have been proposed to characterize the epileptogenicity of the monitored brain structures (Bartolomei et al., 2008; David et al., 2011; Gnatovskyetal., 2011, 2014; Andrzejaketal., 2015; Vila-Vidalet al., "Detection of recurrent activation patterns across focal seizures: Application to seizure onset zone identification",CLINICAL NEUROPHYSIOLOGY, ELSEVIER, AMSTERDAM, NL, vol. 128, no. 6, 3 April 2017 (2017-04-03), pages 977-985, ISSN: 1388-2457, DOI: 10.1016/ J.CLINPH.2017.03.040). These biomarkers quantify preselected spectral features of the iEEG signals in order to assess the degree of involvement of each region in the seizure process. Although the SOZ might be indirectly delineated based on the epileptogenicity of each brain structure, none of the cited studies explicitly aims to design or implement fully unsupervised algorithms. More recent studies have proposed automatic methods based on high-frequency oscillations (HFOs) in interictal or peri-ictal periods, or stochastic properties of the iEEG signals in predefined frequency windows (Geertsema et al. 2015; Liu et al., 2016; Murphy et al., 2017; Varatharajah et al., 2017).

There are also known some patents or patent applications in this field.

US 9326698-B2 discloses a method that detects oscillatory signals representative of discrete events in a patient's body. The detected signals may be tested in the context of surrounding background activity to identify anomalous discrete physiological events that are sufficiently different from the surrounding background activity. The anomalous discrete physiological events having correlative morphological, time, or location characteristics may be automatically clustered and clusters of anomalous physiological events may be determined that are indicative of at least one region of the patient's body that is associated with a medical condition.

US 6678548-B1 discloses a method for predicting and detecting epileptic seizure onsets within a unified multiresolution probabilistic framework, enabling a portion of the device to automatically deliver a progression of multiple therapies, ranging from benign to aggressive as the probabilities of seizure warrant. Based on novel computational intelligence algorithms, a realistic posterior probability function $P(St|x)$ representing the probability of one or more seizures starting within the next T minutes, given observations x derived from IEEG or other signals, is periodically synthesized for a plurality of prediction time horizons. When coupled with optimally determined thresholds for alarm or therapy activation, probabilities defined in this manner provide anticipatory timelocalization of events in a synergistic logarithmic-like array of time resolutions, thus effectively circumventing the performance vs. prediction-horizon tradeoff of singleresolution systems. The longer and shorter prediction time scales are made to correspond to benign and aggressive therapies respectively. The imminence of seizure events serves to modulate the dosage and other parameters of treatment during openloop or feedback control of seizures once activation is triggered. Fast seizure onset detection is unified within the framework as a degenerate form of prediction at the shortest, or even negative, time horizon.

US 2012245481-A1 discloses a method for automatically identifying discrete physiological events such as high-frequency oscillations within the human body and classifying such events for diagnostic purposes. The method can detect and classify oscillatory signals representative of discrete events in a patient's body using a high sensitivity, low specificity detector. The detected signals may be tested in the context of surrounding background activity to identify anomalous discrete physiologic events that are sufficiently different from the surrounding background activity. The anomalous discrete physiologic events may be automatically clustered into clusters of anomalous physiologic events having correlative morphological, time, or location characteristics. At least one cluster of anomalous physiologic events may be determined that is indicative of at least one region of the patient's body that is associated with a medical condition.

Other methods are known by US 2016045127-A1, US 2016287118-A1, WO 2018005981-A1 and US 6594524-B2.

In most of the aforementioned studies or patents, the proposed biomarkers are critically built around spectral features that are confined to predefined frequency bands (either high frequency or whole-spectrum) and might fall short to capture patient-specific seizure onset patterns. Yet, there is no adaptive algorithm that extracts the most relevant features for SOZ localization before proceeding to the localization itself. In addition, these biomarkers are typically applied individually to each signal leveraging on its time samples (e.g. US 9326698-B2, US 2012245481-A1) in contrast to a biomarker that outputs a ∫decision relying on all signals' time samples simultaneously.

Hence, and in a more general fashion, there is a need to develop methodological tools that are capable of extracting the time-frequency features of physiological signals associated with the occurrence of a pre-defined physiological event and jointly detect the relevant physiologic signals that most prominently manifest these patterns using all signals' available information simultaneously. These tools could then be used to determine the brain sites underlying a pathological or a cognitive phenomenon in a patient- and event-specific context.

DESCRIPTION OF THE INVENTION

To that end, embodiments of the present invention provide, according to a first aspect, a computer implemented method for identifying time-frequency features of physiological events, as recited in claim 1. The proposed method comprises receiving, by a computing system having at least one memory and one or more processors, a time period in which a physiological event occurred; a set of physiological signals associated with said physiological event, each signal of the set corresponding to a different spatial location of a body part of a living being either a human or an animal; a time-frequency region of interest; and a plurality of time-frequency windows defined on the time-frequency region of interest.

The cited time-frequency region is defined by a minimum and a maximum time instant and a minimum and a maximum frequency, wherein said minimum and maximum time instants are comprised within said time period in which the physiological event occurred and said maximum frequency is lower or equal than the sampling rate of the physiological signals (for example, lower or equal than the Nyquist frequency of the physiological signals).

Once received the above data, the computing system comprises filtering the set of physiological signals within each of said plurality of time-frequency windows, obtaining as a result a filtered set of physiological signals for each time-frequency window. Then, the computing system calculates, for each defined time-frequency window, a given feature for the filtered set of physiological signals, each one of the signals having a given feature value, providing for each time-frequency window a set of feature values. For each time-frequency window, the computing system then also calculates a first quantifier defined as a function of the set of feature values and/or a second quantifier defined as a function of an empirical distribution of the set of feature values.

If the first quantifier is calculated, the first quantifier is then compared with a given first threshold. In this case, the computer system may select the time-frequency windows satisfying the first threshold (i.e. being either above or below the threshold). If the second quantifier is calculated, this second quantifier is compared with a given second threshold. The computing system then can select the time-frequency windows satisfying the second threshold. Particularly, the first and second thresholds are different.

According to the present invention, the physiological event may be an epileptic seizure, the pre-ictal preparation phase, the brain response to a delivered cognitive or electrical stimulus, the brain response to the administration of a drug, etc. The physiological signals may particularly comprise intracranial electroencephalography (iEEG) signals.

According to an embodiment, the given feature defines an intrinsic attribute of each signal of the filtered set of physiological signals. The intrinsic attribute can include the power in band (PIB) of each signal within each time-frequency window or the mean activation (MA). Particularly, the MA is defined as the instantaneous activation of each signal averaged within each time-frequency window, where said instantaneous activation is the continuous power (for instance, obtained via the Hilbert transform) expressed as a z-score with respect to a common pre-ictal baseline distribution defined by pooling together all signals' power values within that band.

In another embodiment, the given feature defines an attribute that indicates how each signal of the filtered set of physiological signals is related with respect to the other signals of the filtered set. For example, the attribute can be computed using a correlation strength measure (for instance, the average Pearson correlation or Mutual Information), a betweenness centrality measure, a node degree measure, among others.

The first quantifier can comprise any of the mean, the standard deviation, the maximum, the global activation (GA), the minimum or the global inactivation (GI) of the set of feature values. The GA is defined as the weighted average of the set of positive feature values, where each feature value is weighted by itself. The GI is defined as the weighted average of the set of negative feature values, where each feature value is weighted by itself.

The second quantifier can comprise the Renyi entropy, the Fisher information or the activation entropy (AE) of an empirical distribution of the set of feature values. The AE is defined as the Shannon entropy of said empirical distribution.

The computer system can further compare the set of feature values with a given third threshold for each time-frequency window satisfying the first and/or second threshold, thus defining, for each time-frequency window satisfying the first and/or second threshold, a subset of the filtered set of physiological signals, called relevant filtered physiological signals; and accumulate all relevant filtered physiological signals across time-frequency windows satisfying the first and/or second threshold, thus defining a subset of the set of physiological signals, called relevant physiological signals.

According to the proposed method, the time-frequency windows may overlap or not with each other. Likewise, the time-frequency windows may have an equal or different width. Even, the time-frequency windows may be nested windows with initial bound fixed at said minimum time instant and with increasing final bound.

For a particular embodiment, the recorded signals are intracranial electroencephalography (iEEG) signals, the physiological event is an epileptic seizure, the given feature is the mean activation (MA), the first and second quantifiers are the global activation (GA) and the activation entropy (AE), respectively, and the first and second thresholds are a lower threshold for GA and an upper threshold for AE, respectively. In this case, both quantifiers are computed and the computer system further comprises identifying the relevant physiological signals as described above. As a result, the time-frequency windows satisfying both the first and the second thresholds yield maximal and spatially confined spectral activations, thus ensuring that propagation has not started and that SOZ contacts can be naturally discriminated from other sites. The selected time-frequency windows reflect the temporospectral features of the ictal onset patterns and the relevant physiological signals identify the seizure focus. In a similar embodiment, the recorded signals can be scalp electroencephalography (EEG) signals and the computing system would detect the ictal onset patterns and the scalp electrodes where the seizure is first manifested.

In other embodiments, the recorded signals are also intracranial electroencephalography (iEEG) signals from humans or animals and the physiological event is the brain response to a delivered stimulus or the brain response to the administration of a drug. The computing system would detect and localize the brain response to these perturbations.

In some embodiments, the thresholds (first, second and/or third) can be automatically learned by using a controlled set of signals were the true SOZ patterns and contacts are known a priori (e.g., they have been extensively validated by clinical variables including post-surgical outcome) and using a machine learning algorithm such as linear discriminant or support-vector machine classifiers with cross-validation, among others.

Other embodiments of the invention provide, according to other aspects, a system and computer program products including instructions embodied in a non-transitory computer readable medium that, when executed by a processor, cause the processor to identify time-frequency features of physiological events, and if desired, the localization of those events.

Hence, the embodiments described herein provide a fully unsupervised (as well as supervised if the thresholds are learned in annotated sets) and automatic methodology for identifying time-frequency features of physiological events, such as the epileptic seizure onset, epileptogenic sites responding to electrical stimulation, brain sites that respond to drug administration, brain sites that respond to any kind of cognitive stimulation during a task paradigm, etc.

The proposed method carries minimal computational costs. Although the method relies on the a priori detection of the physiological event (e.g. the seizure onset time defined by the clinical neurophysiologist), it needs no information about the frequency and temporal windows of interest, two parameters that are automatically extracted from the spectral properties of the signal. Finally, and more importantly, the achieved automatization of the method does not come at the expense of interpretability. The output of the analysis (the time-frequency windows and the relevant physiological signals) can be easily understood as describing the spectral properties (characteristic frequency, duration and spatial localization) of an electrophysiological pattern during an event of interest. In the context of epilepsy diagnostics, the proposed procedure is particularly suitable to be used as a complementary tool during the pre-surgical evaluation and planning that might help better identify and interpret the regions involved in seizure generation and propagation.

## BRIEF DESCRIPTION OF THE DRAWINGS

The previous and other advantages and features will be more fully understood from the following detailed description of embodiments, with reference to the attached figures, which must be considered in an illustrative and non-limiting manner, in which:

Fig. 1 is a flow chart illustrating a method for identifying time-frequency features of physiological events according to an embodiment of the present invention.

Figs 2A-2C graphically illustrate an embodiment of the method for identifying time-frequency features of physiological events.

Figs. 3A-3C is an exploration of spectral activations in different time-frequency windows of interest done around seizure onset.

Fig. 4 illustrates the processing steps from iEEG signals to SOZ detection.

Figs. 5A-5B illustrate the processing steps of an embodiment of the method to identify spectral changes in brain recordings elicited by the administration of a drug.

DETAILED DESCRIPTION OF PREFERRED EMBODIMENTS

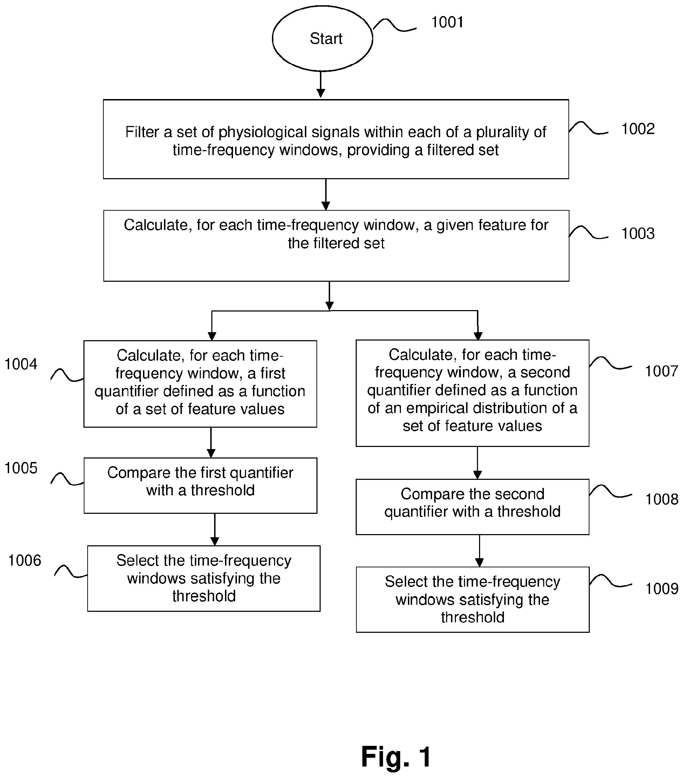

Fig. 1 illustrates a general overview of a method for identifying time-frequency features of physiological events. The method is implemented/executed by a computer and is applied to a set of physiological signals associated with a physiological event, where each signal of said set corresponds to a different spatial location of a body part of a living being, either human or animal.

The general purpose of the method is: 1) to identify the characteristic time and frequency scale of spectral changes in said physiological signals associated with (preceding, following and/or co-occurring with) a particular event for which the time boundaries are (approximately) known, and 2) if desired, to determine its spatial localization, i.e., the region where it occurred. In a particular embodiment as will be detailed later, 1) to identify the spectral properties of ictal onset patterns in electrical signals from the brain upon seizure onset (SO), and 2) when seizure onset is spatially confined, to identify the seizure onset zone (SOZ). In other embodiments, as will be detailed later, 1) to identify the spectral features of patterns elicited in brain electrical signals by the administration of a drug or by localized direct electrical stimulation; and 2) to identify brain regions affected by those changes.

To that end, besides the physiological signals, as input to the method there is also needed a time period in which the physiological event occurred; a time-frequency region of interest defined by a minimum and a maximum time instant comprised within the time period in which the physiological event occurred and a minimum and a maximum frequency, the latter being limited by each signal's sampling rate (in particular, being lower than or equal to the Nyquist frequency of the physiological signals); and a plurality of time-frequency windows defined on the time-frequency region of interest.

It should be noted that the plurality of time-frequency windows may overlap or not with each other and may have equal or unequal widths. Moreover, in some cases it might be preferable to use nested time-frequency windows to quantify the accumulation of activity, i.e. windows with initial bound fixed at the cited minimum time instant and with increasing final bound.

Referring back to Fig. 1, at step 1002, the computer filters the set of physiological signals within each of the time-frequency windows, obtaining a filtered set of physiological signals for each time-frequency window as a result. At step 1003, the computer calculates, for each defined time-frequency window, a given feature for the filtered set of physiological signals; where each signal of the filtered set has a given feature value, providing for each time-frequency window a set of feature values. For example, in an embodiment, the given feature is an intrinsic attribute of each signal of the filtered set of physiological signals, including the power in band (PIB) or the mean activation (MA) of each signal within each time-frequency window. Particularly, the MA is defined as the instantaneous activation of each signal averaged within each time-frequency window, where said instantaneous activation is the continuous power (for instance, obtained via the Hilbert transform) expressed as a z-score with respect to a common pre-ictal baseline distribution defined by pooling together all signals' power values within that band. The PIB can be computed using the Hilbert transform method in narrow bands, the wavelet transform, or the Fourier transform. In another embodiment, the given feature defines an attribute that indicates how each signal of the filtered set is related with regard to the other signals of the filtered set. For example, network measures such as node strength or network centrality built upon within-frequency couplings or cross-frequency coupling can be used.

Then, the computer for each time-frequency window can calculate a first quantifier defined as a function of the set of feature values (step 1004), such as the mean, the standard deviation, the maximum, the global activation (GA), the minimum or the global inactivation (GI). Particularly, the GA and GI target the largest activations and inactivations in the time-frequency windows, respectively, as will be detailed later. Additionally, for each time-frequency window the computer can calculate a second quantifier, defined as a function of an empirical distribution of the set of feature values (step 1007) such as the Fisher information, the Renyi entropy or the activation entropy (AE), that quantifies the structure or confinement of the set of feature values in the time-frequency windows. It should be noted that in other embodiments both first and second quantifiers can be calculated (i.e. both steps 1004 and 1007 can be executed).

After step 1004, the calculated first quantifier is compared, step 1005, with a first threshold. Finally, at step 1006, the computer selects only those time-frequency windows satisfying the threshold. The same applies for the second quantifier, as shown at steps 1008 and 1009.

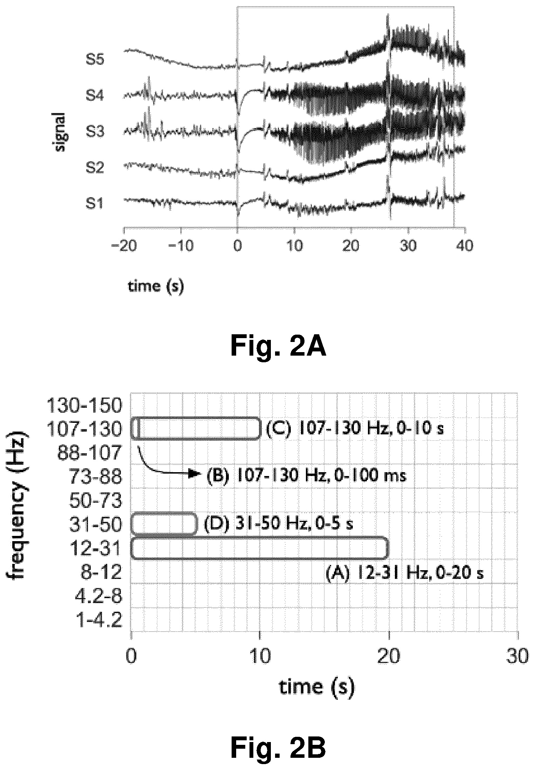

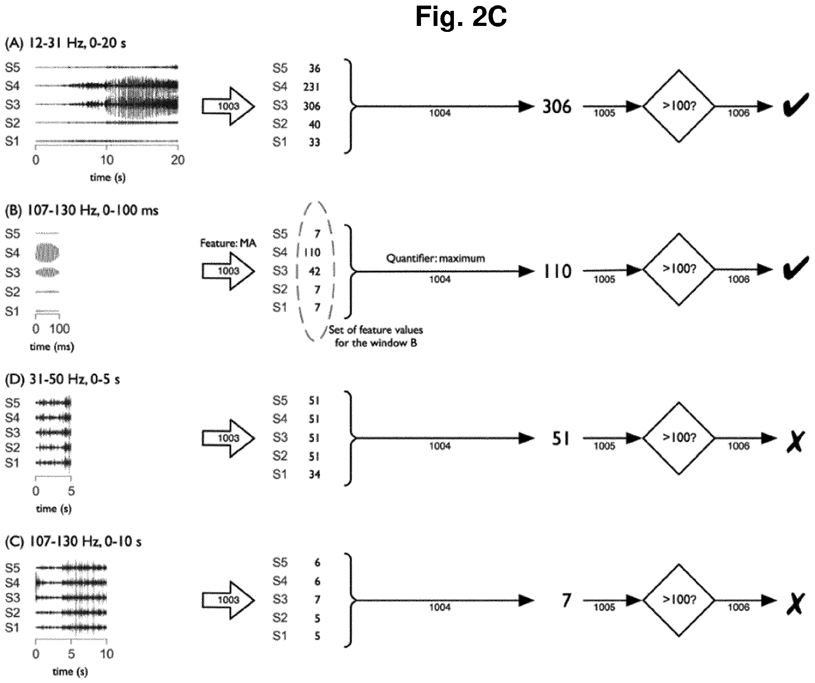

With reference to Figs 2A-2C, therein an embodiment of the proposed method is graphically shown. Fig. 2A graphically depicts (1) a set of physiological signals obtained from 5 sensors; and (2) the time period (highlighted box) in which the physiological event occurred, in this case 0-38 seconds. Fig. 2B illustrates (3) the time-frequency region of interest defined by the user (0-30 seconds, 1-150 Hz); and (4) the plurality of time-frequency windows defined on the time-frequency region of interest (nested windows; 4 exemplary windows are shown as highlighted boxes). On the left-side column (or bottom part) of Fig. 2C the different signals filtered within each time-frequency window are illustrated. On the middle column then it is illustrated how the given feature of each one of the filtered signals is calculated. In this example, the given feature is the MA. For each signal, the MA is defined as the instantaneous activation averaged within each time-frequency window, where said instantaneous activation is the continuous power (for instance, obtained via the Hilbert transform) expressed as a z-score with respect to a common pre-ictal baseline distribution defined by pooling together all signals' power values within that band. On the right-side column (or upper part) of Fig. 2C, it is shown how the quantifier for each window is calculated and compared with a threshold. In this example, the quantifier used is the maximum of all feature values within each window and a common threshold of 100 is used. Windows with max(MA) > 100 are selected. The window selection reveals that the physiological event defined by the highlighted box in Fig. 2A is characterized by initial HFOs (107-130 Hz) lasting 100 ms (invisible to the naked eye). This pattern of HFOs disappears when considering longer time periods (0-10 s). The window selection also reveals that the event is characterized by a prominent pattern of discharges in the beta band (12-31 Hz) at a longer timescale of appearance (0-20 s). Note that, in this example, time-frequency windows in the beta band (12-31 Hz) and time spans around 0-5 s were not selected by the system (these windows are not shown in the figure), reflecting that patterns in the beta band appear progressively some seconds after the onset of the event (compare with Fig. 2A).

Application to Intracranial EEG for Seizure Focus Detection:

Following, a particular embodiment of the proposed method for seizure onset pattern identification and epileptic focus (seizure onset zone, SOZ) prediction will be detailed. This is of particular importance in patients with drug-resistant epilepsy, as the accurate localization of the SOZ is crucial to plan a successful surgery. In this embodiment, the physiological event of interest is the epileptic seizure (and in particular the seizure onset) and the physiological signals are EEG recordings acquired from intracranial electrodes. The system is provided with the seizure onset time, and with a time-frequency region of interest around this time, with the plurality of time-frequency windows to be explored.

To test the method, ten patients with pharmacoresistant epilepsy were selected. These patients underwent stereotactic-EEG presurgical diagnosis at the Epilepsy Unit from Hospital del Mar, Barcelona, Spain. As part of the validation protocol, the EEG epochs containing a total of 67 seizures were selected, annotated and documented by trained epileptologists from this unit and its associated Epilepsy Research Group, within the Neurosciences program at the Hospital del Mar Medical Research Institute (IMIM), Barcelona, Spain. Seizure onset and termination times of each seizure were independently marked by two epileptologists.

Patient inclusion was based on the following criteria: a) that the seizure focus had been identified by the epileptologists and b) that ictal onset was confined to a reduced number of contacts. After electrode implantation and monitoring, SOZ was identified by neurologists using visual inspection. Surgical resection or radio-frequency thermocoagulation (RFTC) was planned based on individual SEEG evaluations. Intracranial EEG recordings were obtained using 5 to 21 intracerebral multiple contact electrodes that were stereotactically implanted using robotic guidance. Signals were recorded using a standard clinical EEG system with 500 Hz of sampling rate, except for patient 3, where a sampling rate of 250 Hz was used

For each seizure the computer selected the marked ictal epoch together with 60 seconds of pre-ictal baseline activity. Artifacted channels were identified by visual inspection and removed prior to data analysis. A band-pass filter (FIR, filter band [1,165] Hz) was used to remove slow drifts and aliasing effects. A notch FIR filter at 50 Hz and its harmonic frequencies was also used to remove the alternate current interference.

Here, the time-frequency region of interest was defined with the following parameters: from 0 to 30 s after seizure onset (SO) and from 1 to 150 Hz. In order to systematically explore different windows for ictal onset pattern identification and for SOZ detection, the following time-frequency grid was defined for exploration. The frequency spectrum was divided into a set of 10 non-overlapping bands. A set of nested time windows obtained by fixing the left bound at SO and varying the right bound were considered. Specifically, the frequency spectrum was splitted into 10 non-overlapping bands using the following cutting points: 1, 4.2, 8, 12, 31, 50, 73, 88, 107, 130 and 150 Hz. In the time-domain, windows with right bound from 100 ms to 30 s after using steps of 100 ms from 100 ms to 5 s and steps of 1 s from 5 to 30 s were considered (i.e., from SO to SO+0.1s, from SO to SO+0.2s, ..., from SO to SO+4.9s, from SO to SO+5s, from SO to SO+6s, ..., from SO to SO+30s).

For each time-frequency window, the mean activation (MA) (Vila-Vidal et al., 2017) of each recorded signal was computed by the system and used as an intrinsic feature of that signal. The MA quantifies the average instantaneous activation of each targeted brain structure for a pre-defined frequency and time windows of interest. The instantaneous activation is the continuous power of each signal (for instance, obtained via the Hilbert transform) in a specific band expressed as a z-score with respect to a single pre-ictal baseline distribution defined by pooling together all signals' power values within that band.

This quantity is estimated from the EEG signals using a two-step procedure, as described in the next paragraph.

Let $x(t)$ denote a single-channel EEG signal, and let's assume that the time-frequency window of interest is defined by the frequency range $[f_1, f_2]$ and by time points $t_1$ and $t_2$. In practice, $x(t)$ is a discrete time-series denoted by $x[k] = x(t), t = k\delta, k = 1, ..., K$, where $\delta$ is the sampling period and $K$ is the total number of samples contained in the recording. Using this notation, time points $t_1$ and $t_2$ are indexed as samples $k_1$ and $k_2$, respectively. In the first step, a time-varying spectral activation in the frequency range of interest is obtained using the Hilbert transform method. The signal $x[k]$ is band-pass filtered in non-overlapping logarithmically-spaced narrow frequency bands $[f, f + \Delta f]$ ($\Delta f = 0.1$ was used) covering the whole frequency range of interest $[f_1, f_2]$. Signal power in each narrow band is obtained by squaring the signal envelope (modulus of the analytical signal). Summation of the signal power over all narrow frequency bands is performed to obtain the time-dependent power of each region's EEG signal in the desired frequency of interest $[f_1, f_2]$ (PIB). The resulting values are z-scored with respect to a baseline distribution defined by the power values of all contacts in a non-ictal epoch (in this case, from 60 to 20 seconds before ictal onset) to obtain an activation time series $A[k]$ that can be interpreted as a measure of the power change from the pre-ictal state at any given point. Note that $A[k]$ can take both positive and negative values. In the second step, activations are averaged in the time window of interest to obtain the MA for each recording site. For the precise estimation of time-averaged activations, artifactinduced noise should be first removed. Time-stamps with frequency-specific artifacts simultaneously affecting the majority of EEG signals are detected with a sliding-window analysis (200 samples width, 1 sample step), performed independently in the pre-ictal and ictal epochs. Time windows where the product of the mean correlation and the contact-average signal power is two standard deviations larger than the median are considered as artifacts and are discarded in the subsequent analysis. Finally, for a given recording site, the mean activation (MA) in the frequency range $[f_1, f_2]$ and in the time period $[t_1, t_2]$ is obtained by averaging the activation $A[k]$ over the time window of interest (Vila-Vidal et al., 2017):

$$\text{MA} = \frac{1}{k_2 - k_1} \sum_{k=k_1}^{k_2} A[k]$$

Applying this procedure to each recorded signal within a given time-frequency window, one obtains a set of MA values, one for each physiological signal. By repeating the computation within each time-frequency window of the time-frequency grid, the computing system obtains a set of MA values for each window. The MA of a given signal $j$ in the frequency band $[f, f + \Delta f]$ will be denoted and computed over a time window spanning from the seizure onset until time $t$ with the following notation: $MA_j(f, t)$.

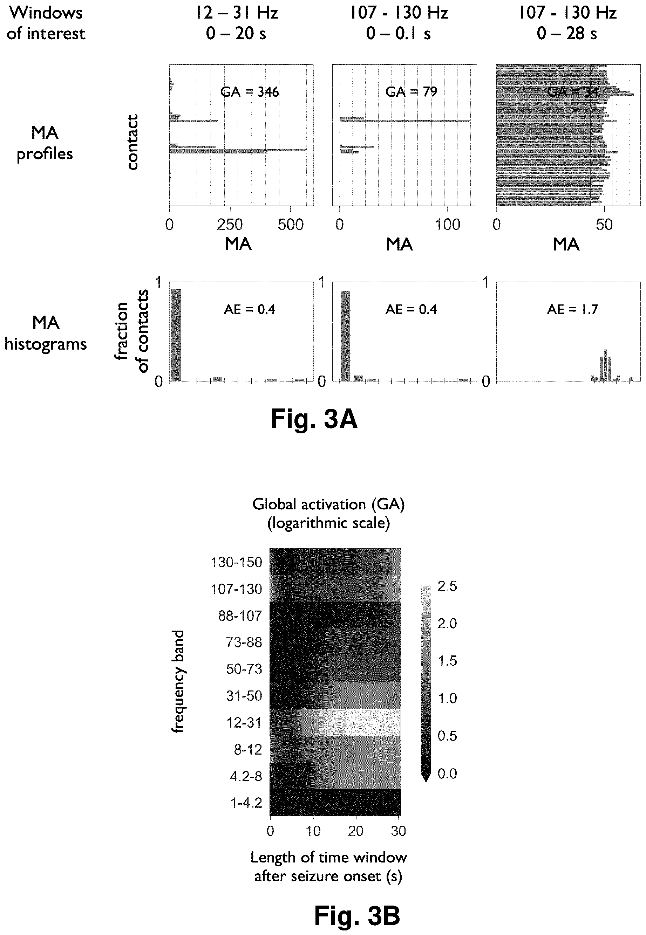

For optimal focus detection it must be ensured that there is a hierarchical and selective activation of SOZ contacts only. Central to the proposed approach is the definition of two new quantifiers, namely the GA (Global Activation) and the AE (Activation Entropy), that in this particular case are jointly optimized to find time-frequency windows of interest where ictal activity is maximal with respect to a baseline pre-ictal period, while being spatially confined to a few contacts. Figs. 3A-3C illustrate how these quantifiers are computed and used to assess the amount of information carried in each window of interest. For each time-frequency window, the GA quantifies the magnitude of the most relevant spectral activations with respect to the pre-ictal basal state. It is defined as the weighted average of the set of MA values over all contacts with positive MA, where each contact's contribution is weighted by its MA value, thus ensuring that most active regions have a higher impact on the final value (Fig. 3A):

$$GA(f, t) = \frac{\sum_{j=1}^{N} w_j * MA_j(f, t)}{\sum_{j=1}^{N} w_j},$$

with

$$w_j = MA_j(f, t) \text{ if } MA_j(f, t) > 0, \text{ and } w_j = 0, \text{ if } MA_j(f, t) \le 0.$$

For each time-frequency window, the AE characterizes the spatial spread of these spectral activations. It is defined as the entropy of an empirical distribution obtained by defining a number of discrete activation ranges on the set of MA values. First, a histogram is computed over the set of MA values using h bins homogeneously spaced between the minimum and maximum MA values (here, h = 10 was used). Probability values for each bin ($p_i$ for $i$ = 1, ...,10) are found as the fraction of contacts lying within the corresponding MA bin (Fig. 3A). The computing system then computes the Shannon's entropy of this empirical distribution using the formula:

$$AE(f,t) = -\sum_{i=1}^{10} p_i * \log p_i\,.$$

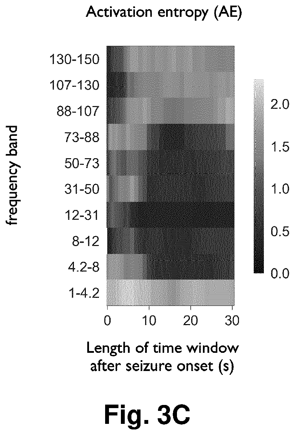

For each time-frequency window in the exploration grid, the computing system extracted the GA and AE quantifiers from the set of MA values. Fig. 3B and 3C show the GA and AE quantifiers for one exemplary seizure, respectively.

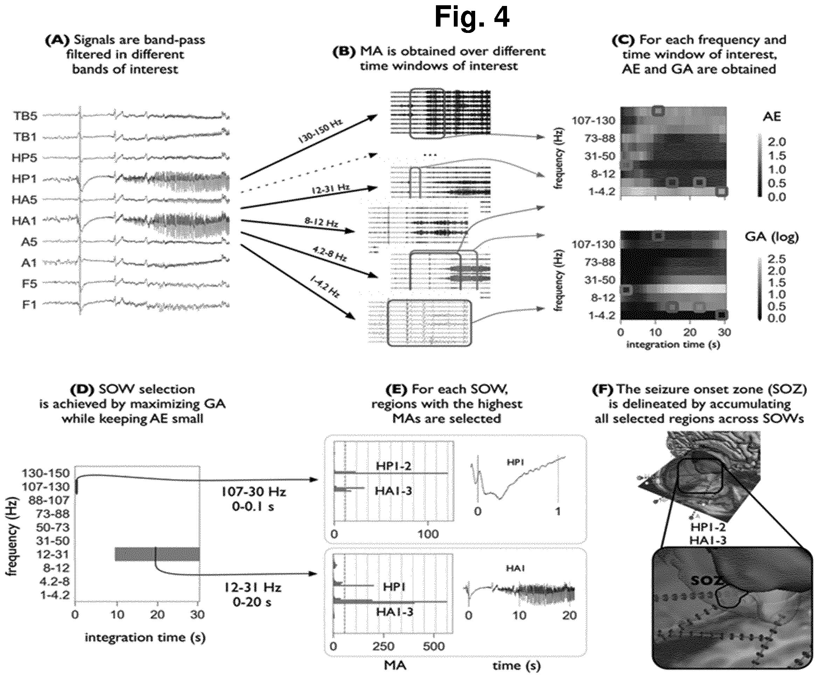

Figs. 4 summarizes the processing steps to obtain the optimal time-frequency windows for SOZ detection (referred to as seizure onset windows, SOWs) and the SOZ. Intracranial EEG signals in the peri-ictal period were band-pass filtered in pre-defined bands of interest spanning the whole spectrum (Fig. 4A). Then, for each band, MAs were obtained for all possible time windows of interest (Fig. 4B). For each time-frequency window in the exploration grid, the computer extracted the GA and AE from the set of MA values (Fig. 4C).

Seizure onset window (SOW) detection was achieved by finding time-frequency windows that maximized the GA under the constraint of low AE to ensure that spectral activations were confined only to a few contacts. All pairs (f, t) were considered by the computing system and two threshold conditions, one per variable, were set. Regarding the first variable, a first or lower threshold was set at the 95-th percentile of the GA distribution. For time-frequency-windows to be considered, they were further required to have a GA above 3 to ensure significant global activations with respect to the pre-ictal state. Additionally, a second or upper threshold of 0.5 was set on the AE. All time-frequency windows satisfying both conditions were preselected as candidates to be SOWs. Finally, for each frequency band the first time windows satisfying the condition were only kept. Finally, for each frequency band only the first set of consecutive time windows fulfilling the required criteria was kept. Fig. 4D shows the selected SOWs for the first seizure of patient 1.

The procedure described before was sequentially applied to the 67 seizures included in the validation. In each seizure, the SOWs were qualitatively found to pinpoint the characteristic frequency and time windows of the seizure onset patterns. As an example, in the first seizure of patient 1, the algorithm selected the following SOWs: 107-130 Hz during the first hundreds of milliseconds and 12-31 Hz between 10 and 20 s. Regions of the posterior and anterior hippocampi were selected as being inside the SOZ. The inspection of the electrophysiological activity around the seizure onset by epileptologists revealed that the output of the method was qualitatively describing the seizure onset patterns. As seen in Fig. 4E the seizure is initiated at a hippocampal level with rapid discharges (~110 Hz) of very low amplitude in the first hundreds of milliseconds after seizure onset, combined with an inverted depolarizing wave. These discharges are particularly clear in HP1 and are followed by a drastic decrease in frequency evolving to low-voltage fast-activity (LVFA) at 12-31 Hz that becomes visible 5 seconds after seizure onset and that increases in amplitude as the seizure progresses. In this case, activations identified at 12-31 Hz are in fact a combined effect of LVFA activity (~30 Hz) together with slow rhythmic spikes (RS) (~15 Hz) of high amplitude particularly observable between 10 and 20 s.

In each SOW, the relevant iEEG channels were found by applying a third threshold on the set of MA values. In this case, the third threshold was induced by the threshold applied on the AE quantifier. An upper threshold of 0.5 on the AE quantifier implies that, for each time-frequency windows satisfying the threshold, at least 80% of the contacts lie within the same MA bin. For each SOW, the computing system identified the highly populated MA bin (having at least 80% of the contacts), which was used to define the third threshold. Contacts above this bin were considered to be part of the SOZ. This procedure was repeated for all selected SOWs, and SOZ contacts were accumulated, thus obtaining a single SOZ per seizure (Fig. 4F).

For each patient, the SOZ was defined by accumulating all seizure-specific detected SOZ regions. Then the SOZ defined by epileptologists was used as the ground truth to assess the performance of the proposed method. For each patient, the sensitivity and specificity of the selection were computed. The average sensitivity of the method across patients was $0.94\pm0.09$ (mean $\pm$ standard deviation), with an average specificity of $0.90\pm0.09$ (mean $\pm$ standard deviation). In 7 patients all SOZ regions were identified by the method (sensitivity=1). In the remaining 3 false negative cases (SOZ contacts mistakenly marked as non-SOZ) lied at most 1 contact (i.e. 1.5 mm) apart from true positives (regions correctly marked as SOZ).

The robustness of the method against particular choices of the thresholds was studied by computing a ROC curve as the thresholds varied. Small fluctuations of the GA threshold (95th percentile) and the AE threshold (0.5) were not found to significantly alter the sensitivity and specificity of the results. As the conditions on GA and AE are relaxed more non-SOZ sites are chosen. Despite being outside the SOZ, it was hypothesized that these sites might have a critical role in sustaining and propagating epileptic activity in the early stages of the seizure.

The amount of SOZ predictability carried in the pre-ictal activity was also assessed. To do so, time windows spanning from seizure onset into the past, i.e., with final bound at seizure onset and initial bound ranging from 30 s to 0.1 s before seizure onset, with the same spacing as before, were also considered. The average sensitivities and specificities of the method across patients in the pre-ictal period were lower than in the ictal period: $0.77 \pm 0.32$ (mean $\pm$ standard deviation) and $0.77 \pm 0.12$, respectively. Although the method has a better performance in the ictal period, high sensitivities and specificities indicate that the pre-ictal period contains sufficient information for SOZ prediction.

Finally, to relate the results of the proposed method with validated post-operative information, patients that underwent either surgery (n=5) or RFTC (n=4) were subselected and the degree of overlap between the predicted SOZ and the treated areas was quantified. Cross-validation with postoperative outcome showed that the fraction of predicted SOZ regions that were treated tended to be higher in patients attaining seizure freedom after a sufficiently long follow-up period that in those exhibiting symptom relapse after the treatment, being this trend higher in surgery than in RFTC. Yet, the proportion of treated SOZ regions lies in the range 0.25-0.85, highlighting the non-trivial relationship between the SOZ and the epileptogenic zone.

The method for seizure onset pattern identification and epileptic focus prediction was also tested with 9 pharmacoresistant epileptic patients admitted at Hospital Clinic for intracranial video-EEG monitoring between January 2017 and March 2019, including patients with extra-temporal lobe epilepsy. These patients underwent stereotactic-EEG presurgical diagnosis at the EEG lab from the Epilepsy Unit in Hospital Clinic, Barcelona, Spain. Intracranial EEG recordings were obtained using intracerebral multiple contact electrodes that were stereotactically implanted using robotic guidance. Signals were recorded using a standard clinical EEG system with 1024 or 2048 Hz of sampling rate.

As part of the validation protocol, the EEG epochs containing a total of 44 seizures were selected, annotated and documented by trained epileptologists from this unit. All seizures were processed by the system using the same configuration and procedure as used with patients from Hospital del Mar without any further adaptation or adjustment. Upon revision with clinicians, the system was qualitatively found to pinpoint the ictal onset patterns in a significant proportion of seizures (73%). A preliminary analysis showed that the system correctly identified the SOZ in 6 of the 9 patients when running blindly.

<u>Application to Scalp EEG for Seizure Focus Detection:</u>

In another embodiment, the method is used to identify ictal onset patterns in scalp EEG and to pinpoint scalp electrodes where those patterns where first registered. In this case, the physiological event of interest is also the onset of an epileptic seizure, but the physiological signals are EEG recordings acquired from scalp electrodes. The present invention was applied for seizure onset pattern identification in scalp EEG recordings. To this end, 2 patients that underwent non-invasive long-term video-EEG monitoring in 2017 in the Epilepsy Unit at Hospital Clinic, Barcelona, Spain, were selected. Video-EEG was performed using a 64-channel Neurolink 64 inbox-1166A amplifier and recorded at 1024 Hz using NeuroWorks software (Natus Medical Inc.). Superficial electrodes were located using the 10/20 International system, using additional electrodes in the frontotemporal regions according the 10/10 system. Video-EEG monitoring was performed in the epilepsy unit for 5 d, and antiepileptic drugs were reduced when necessary to facilitate seizure occurrence.

EEG epochs containing a total of 4 seizures (2 per patient) were selected, annotated and documented by trained epileptologists from the unit. All seizures were processed by the system using the same configuration and procedure as used with intracranial EEG, except for the AE threshold that was set to 0.7. In all cases, the system identified initial gamma rhythms upon seizure onset that lasted for some seconds before propagation. In particular, the system showed that in some cases HFOs (>130 Hz) that had not been identified in visual inspection by the clinicians were also present in the first milliseconds after seizure onset.

<u>Application to Intracranial EEG for Detection of Brain Responses Evoked by Direct Electrical Stimulation:</u>

In another embodiment, the system is used to detect spectral patterns elicited by electrical stimulation applied with invasive or non-invasive electrodes and to identify the contacts where these patterns occur. To test this embodiment, intracranial EEG data collected from an epileptic patient that underwent a session of direct electrical stimulation as part of his/her pre-surgical diagnosis at the Epilepsy Unit of Hospital del Mar, Barcelona, was used. In this analyzed patient, seven electrodes were implanted with a total of 80 channels. The sampling rate of the EEG data was 500 Hz. Specifically, the method was applied to analyze the post-stimulation effect that an electrical stimulation delivered on a specific pair of channels in the frontal cingulate area, and which lasted 25 seconds, had on all recorded channels.

Artifacted channels were identified by visual inspection and removed prior to data analysis. After removal of the stimulation period, a band-pass filter (FIR, filter band [1,165] Hz) was used to remove slow drifts and aliasing effects in the pre-stimulation and post-stimulations periods independently. A notch FIR filter at 50 Hz and its harmonic frequencies was also used to remove the alternate current interference. The system was fed with the EEG signals. In this case, the event of interest was defined as the response to the electrical stimulation during 30 seconds. Here, the same time-frequency region of interest and plurality of time-frequency windows as in the embodiment described for SOZ detection with patients from Hospital del Mar were used. Specifically, the time-frequency region of interest was defined with the following parameters: from 0 to 30 s after stimulation offset (SO) and from 1 to 150 Hz. The frequency spectrum was divided into a set of 10 non-overlapping bands. A set of nested time windows obtained by fixing the left bound at SO and varying the right bound were considered. Specifically, the frequency spectrum was splitted into 10 non-overlapping bands using the following cutting points: 1, 4.2, 8, 12, 31, 50, 73, 88, 107, 130 and 150 Hz. In the time-domain, windows with right bound from 100 ms to 30 s after stimulation offset (SO) using steps of 100 ms from 100 ms to 5 s and steps of 1 s from 5 to 30 s were considered (i.e., from SO to SO+0.1s, from SO to SO+0.2s, ..., from SO to SO+4.9s, from SO to SO+5s, from SO to SO+6s, ... , from SO to SO+30s).

In this case, the feature used to characterize each EEG signal within each time-frequency window was the average power of each signal in a specific band z-scored with respect to the signal's baseline statistics (i.e., data was demeaned by the baseline mean and then normalized by the baseline standard deviation). As a baseline, the system used a 5-seconds artifact-free pre-stimulation period. This quantity was estimated using the following procedure.

For each signal and frequency band of interest, the system estimated the time-dependent power using the Hilbert transform. Then, resulting values of each contact were z-scored with respect to baseline statistics for each contact and frequency band independently. Then, for each time-frequency window of interest, the system computed the average z-scored power over the time-window of interest.

For each time-frequency window, the system computed the GA and selected windows with GA>3, searching for increases in power. The system reported general increases in delta, theta, alpha and beta waves (1-30 Hz) that lasted around 30 seconds after stimulation offset. No effect was found in higher frequencies. Additionally, the system computed the AE and selected windows with GA>3 and AE<0.6, searching for spatially confined activations elicited by the stimulation. The system reported an increase in alpha (8-12 Hz) that lasted around 15 seconds and that was visible not only in the frontal cingulate area, but also in distant contacts from the temporal gyri, pinpointing possible propagation paths through fiber tracts.

Application to Intracranial Local Field Potentials for Detection of Brain Responses Evoked by Drug Administration:

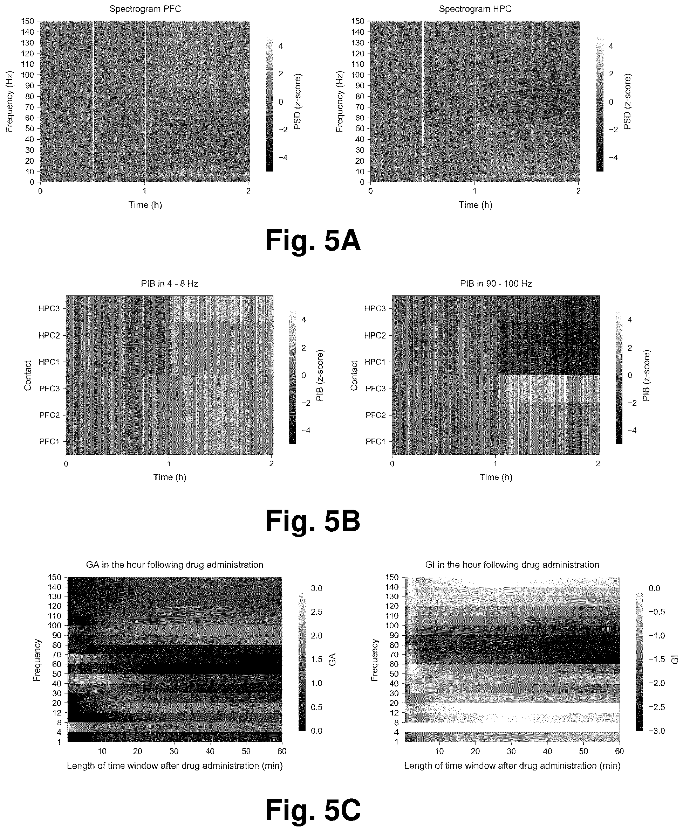

Following, another embodiment of the proposed technology to identify spectral changes in brain recordings elicited by the administration of a drug will be detailed (illustrated in Figs. 5A-5C). In this embodiment, the physiological event of interest is the administration of the drug and the physiological signals are EEG recordings acquired from scalp or intracranial electrodes. The system is provided with the time period where the drug is infused, with a time-frequency region of interest after this time, with a plurality of time-frequency windows to be explored.

In particular, the method was tested in intracranial electrophysiological data from a freelymoving mouse during a pharmacological experiment reported in a previous publication (Gener et al., 2019). The experimental paradigm consisted of 30min baseline period, 30 min following administration of saline, 1 h following administration of the first drug (agonists and antagonists), and 1 h period following administration of the second drug (antagonists following agonists only). During each experiment, intracranial recordings sampled at 30Khz were obtained from several electrodes implanted in the prefrontal cortex (PFC) and hippocampus (HPC). A complete description of the experimental design and data recordings is provided in (Gener et al., 2019). To test the detector, the PFC and HPC local field potential (LFPs) were reused in one mouse following the administration of 1 mg of 1-(2,5-dimethoxy-4-iodophenyl)-2 amino propane (DOI, partial 5-HT2A/2CR agonist) as a first drug. In total 3 contacts from the PFC and 3 contacts from the HPC were selected. To obtain LFPs, signals were downsampled to 1 kHz, detrended and notch-filtered to remove noise line artifacts (50 and 100 Hz) with customwritten scripts in Python. A band-pass filter (FIR, filter band [1,250] Hz) was used to remove slow drifts and aliasing effects. As part of the preprocessing, signals were also normalized using the z-score.

In order to detect the time-frequency scale where changes occurred following the drug administration, a set of time-frequency windows were defined. The frequency spectrum was divided into a set of 17 non-overlapping bands using the following cutting points: 1, 4, 8, 12, 20, 30, 40, 50, 60, 70, 80, 90, 100, 110, 120, 130, 140 and 150 Hz. In the time-domain, a set of nested time windows obtained by fixing the left bound at drug administration and varying the right bound were considered. A total of 120 time-windows with right bound from 30 s to 1 h after drug administration (DA) were considered, increasing the window size in steps of 30 s (i.e., from DA to DA+30 s, from DA to DA+60s, from DA to DA+90s, ..., from DA to DA+1h). In this case, the feature used to characterize each LFP signal within each time-frequency window was the average power in band (PIB) normalized with respect to the baseline PIB distribution of that signal in that particular frequency band. This quantity was estimated using a slicing window and a multi-taper method as described in the next paragraph.

For each signal, a spectrogram from 1 to 150 Hz was constructed using a slicing window and a multi-taper method. Slicing windows (not to be confused with time windows of interest) with window step and window length equal to 30 s were used. Within each slicing window, power spectral density (PSD) from 1 to 150 Hz was estimated using discrete prolate spheroidal sequences (DPSS) with standardized half bandwidth set to 5 and orders ranging from 1 to 9 (a total of 9 tapers). Fig. 5A shows the spectrograms for two signals from PFC and HPC. Then, for each frequency band defined above, the PIB was obtained by summating the spectrogram over the frequencies contained in that band. This resulted in a time-dependent PIB per signal and frequency band defined above for the whole recording (sampled at 1/30 Hz). For each channel and frequency band, the PIB time-course was z-scored with respect to baseline statistics (i.e., data was demeaned by the baseline mean and then normalized by the baseline standard deviation). As such, the z-scored PIB reflects the power change of a given signal in a given frequency band elicited by the administration of a drug, with positive (resp. negative) values reflecting increases (resp. decreases) in power with respect to the baseline activity of that contact. Fig. 5B shows the z-scored PIB time-courses for two exemplary bands showing spectral changes upon drug administration (4-8 Hz and 90-100 Hz). Finally, for a given time-frequency window defined in the previous paragraph, the feature of the set of LFP signals was obtained by averaging the z-scored PIB values over the time windows of interest. Note that, with a sampling of 1 PIB value every 30 s, the i-th time window of interest (from DA to DA+ 30i) contains a total of i PIB samples.

Applying this procedure to each recorded signal within a given time-frequency window, one obtains a set of average z-scored PIB values, one for each physiological signal. By repeating the computation within each time-frequency window of the time-frequency grid, the computing system obtains a set of PIB values for each window. For the sake of clarity, the average z-scored PIB value of a given signal j in the frequency band $[f, f + \Delta f]$ and computed over a time window spanning from DA until time $t$ is denoted with the following notation: $Z_j(f,t)$.

For each time-frequency window, two different versions of the method were used. In the first version, the GA (defined above) computed over Z as the first quantifier was used. When computed over Z, the GA is the average of the set of Z values over all contacts with positive Z, where each contact's contribution is weighted by the magnitude of its own Z value, thus ensuring that signals with a most prominent increase in power have a higher impact on the final value:

$$GA(f,t) = \frac{\sum_{j=1}^{N} w_j * Z_j(f,t)}{\sum_{j=1}^{N} w_j},$$

with

$$w_j = Z_j(f,t) \text{ if } Z_j(f,t) > 0, \text{ and } w_j = 0, \text{ if } Z_j(f,t) \leq 0.$$

Then, time-frequency windows with GA > 1.24 (3rd quantile of the GA distribution) were selected. Within each window satisfying this condition, the method further identified contacts responsible for this global increase in power by applying a threshold on the Z values. Contacts with with Z > 1.24 were selected.

For the second version, the GI (Global Inactivation) was introduced, a new first quantifier to compute over Z. The GI is defined as the weighted average of the set of Z values over all contacts with negative Z, where each contact's contribution is weighted by the magnitude of its own Z value, thus ensuring that signals with a most prominent decrease in power have a higher impact on the final value:

$$GI(f,t) = \frac{\sum_{j=1}^{N} w_j * Z_j(f,t)}{\sum_{j=1}^{N} w_j},$$

with

$$w_j = Z_j(f,t) \text{ if } Z_j(f,t) < 0, \text{ and } w_j = 0, \text{ if } Z_j(f,t) \geq 0.$$

In this case, time-frequency windows with a GI < -1.7 (1st quantile of the GI distribution) were selected. Within each window satisfying this condition, the method further identified contacts responsible for this global decrease in power by applying a threshold on the Z values. Contacts with Z < -1.7 were selected.

Fig. 5C shows the quantifiers used in the two different versions of the system: the Global Activation (GA) targets the most prominent increases in PIB regardless of the contact where they occur, while the Global Inactivation (GI) targets the most prominent decreases in PIB regardless of the contact where they occur. Drug administration occurs at 0 min.

Using this procedure, the system reported the following results. Following drug administration, an increase in power in the band 4-8 Hz was observed in some contacts both of PFC and HPC that lasted until the end of the experiment. Additionally, an increase in 40-50 Hz was reported in HPC during the first 20 minutes after DA, that later evolved to a slower rhythm at 12-20 Hz until the end of the experiment.

Significantly, a power increase in the gamma range (80-110 Hz) was reported in one contact of the PFC, starting 20 minutes after DA and lasting until the end of the recording, in line with the results described in the research article. This power increase in PFC was accompanied by a power decrease in HPC starting 5-10 minutes after DA and lasting until the end of the recording.

The present invention has been described in particular detail with respect to specific possible embodiments. Those of skill in the art will appreciate that the invention may be practiced in other embodiments. Further, the system and/or functionality of the invention may be implemented via various combinations of software and hardware, as described, or entirely in software elements. Also, particular divisions of functionality between the various components described herein are merely exemplary, and not mandatory or significant. Consequently, functions performed by a single component may, in other embodiments, be performed by multiple components, and functions performed by multiple components may, in other embodiments, be performed by a single component.

Certain aspects of the present invention include process steps or operations and instructions described herein in an algorithmic and/or algorithmic-like form. It should be noted that the process steps and/or operations and instructions of the present invention can be embodied in software, firmware, and/or hardware, and when embodied in software, can be downloaded to reside on and be operated from different platforms used by real-time network operating systems.

The scope of the present invention is defined in the following set of claims.

References

Andrzejak RG, David O, Gnatkovsky V, Wendling F, Bartolomei F, Francione S, et al. Localization of epileptogenic zone on pre-surgical intracranial EEG recordings: toward a validation of quantitative signal analysis approaches. Brain Topography 2015; 28(6): 832-837.

Bartolomei F, Chauvel P, Wendling F. Epileptogenicity of brain structures in human temporal lobe epilepsy: a quantified study from intracerebral EEG. Brain 2008; 131 (7): 1818-1830.

David O, Blauwblomme T, Job AS, Chabardès S, Hoffmann D, Minotti L, et al. Imaging the seizure onset zone with stereo-electroencephalography. Brain 2011; 134(10): 2898-2911.

Geertsema EE, Visser GH, Velis DN, Claus SP, Zijlmans M, Kalitzin SN. Automated seizure onset zone approximation based on nonharmonic high-frequency oscillations in human interictal intracranial eegs. International Journal of Neural Systems 2015, 25(05): 1550015.

Gener T, Tauste Campo A, Alemany-González M, Nebot P, Delgado-Salient C, Chanovas J, Puig MV. Serotonin 5-HT1A, 5-HT2A and dopamine D2 receptors strongly influence prefronto-hippocampal neural networks in alert mice: Contribution to the actions of risperidone Neuropharmacology 2019; 158: 107743. https://doi.org/10.1016/j.neuropharm.2019.107743

Gnatkovsky V, Francione S, Cardinale F, Mai R, Tassi L, Lo Russo G, et al. Identification of reproducible ictal patterns based on quantified frequency analysis of intracranial EEG signals. Epilepsia 2011; 52(3): 477-488.

Gnatkovsky V, de Curtis M, Pastori C, Cardinale F, Lo Russo G, Mai R, et al. Biomarkers of epileptogenic zone defined by quantified stereo-EEG analysis. Epilepsia 2014; 55(2): 296-305.

Liu S, Sha Z, Sencer A, Aydoseli A, Bebek N, Abosch A, et al. Exploring the time-frequency content of high frequency oscillations for automated identification of seizure onset zone in epilepsy. Journal of Neural Engineering 2016; 13(2): 026026.

Murphy PM, von Paternos AJ, Santaniello S. A novel HFO-based method for unsupervised localization of the seizure onset zone in drug-resistant epilepsy. In: Engineering in Medicine and Biology Society (EMBC), 2017 39th Annual International Conference of the IEEE. IEEE, 2017. P. 1054-1057.

Varatharajah Y, Berry BM, Kalbarczyk ZT, Brinkmann BH, Worrell GA, and Iyer RK. Inter-ictal seizure onset zone localization using unsupervised clustering and bayesian filtering. In: Neural Engineering (NER), 2017 8th International IEEE/EMBS Conference on. IEEE, 2017. P. 533-539.

Vila-Vidal M, Principe A, Ley M, Deco G, Tauste Campo A, Rocamora R. Detection of recurrent activation patterns across focal seizures: Application to seizure onset zone identification. Clinical Neurophysiology 2017; 128(6): 977-985.

Claims

1. A computer implemented method for identifying time-frequency features of physiological events, the method comprising:

a) receiving, by a computing system:

a time period in which a physiological event occurred;
a set of physiological signals associated with said physiological event, each signal of said set of physiological signals corresponding to a different spatial location of a body part of a living being;
a time-frequency region of interest, said time-frequency region being defined by a minimum and a maximum time instant and a minimum and a maximum frequency, wherein said minimum and maximum time instants are comprised within said time period in which the physiological event occurred and said maximum frequency is lower or equal than the sampling rate of the physiological signals; and
a plurality of time-frequency windows defined on the time-frequency region of interest;

b) filtering the set of physiological signals within each of said plurality of time-frequency windows, obtaining as a result a filtered set of physiological signals for each time-frequency window;
c) calculating, by the computing system, for each defined time-frequency window, a given feature for the filtered set of physiological signals, each one of the signals of the filtered set of physiological signals having a given feature value, providing for each time-frequency window a set of feature values; and
d) calculating, by the computing system, for each time-frequency window, at least one of:

a first quantifier defined as a function of said set of features values and comparing the calculated first quantifier with a given first threshold, the computing system further selecting the time-frequency windows satisfying the first threshold; and/or
a second quantifier defined as a function of an empirical distribution of said set of feature values and comparing the calculated second quantifier with a given second threshold, the computing system further selecting the time-frequency windows satisfying the second threshold.

2. The method of claim 1, wherein said given feature calculated in step c) defines an intrinsic attribute of each signal of the filtered set of physiological signals, the intrinsic attribute including at least one of the power in band (PIB) of each signal within each time-frequency window or the mean activation (MA), defined as the instantaneous activation of each signal averaged within each time-frequency window, said instantaneous activation being a continuous power expressed as a z-score with respect to a common pre-ictal baseline distribution defined by pooling together all signals' power values within that band.

3. The method of claim 1, wherein said given feature in step c) defines an attribute that indicates how each signal of the filtered set of physiological signals is related with respect to the other signals of the filtered set, said attribute including at least one of the average Pearson correlation, the average Mutual Information, the betweenness centrality of each signal with respect to all other signals within each time-frequency window.

4. The method of any of previous claims, wherein the first quantifier comprises at least one of the mean, the standard deviation, the maximum, a global activation, GA, the minimum, or a global inactivation, GI, of the set of feature values, wherein the GA is defined as the weighted average of the set of positive feature values, where each feature value is weighted by itself, and wherein the GI is defined as the weighted average of the set of negative feature values, where each feature value is weighted by itself.

5. The method of any of previous claims, wherein the second quantifier comprises at least one of the Renyi entropy, the Fisher information or an activation entropy, AE, of an empirical distribution of the set of feature values, wherein the AE is defined as the Shannon entropy of said empirical distribution.

6. The method of any of previous claims 1 to 3, wherein the first quantifier comprises a global activation, GA, a measure defined as the weighted average of the set of positive feature values, where each feature value is weighted by itself, and wherein the second quantifier comprises an activation entropy, AE, a measure defined as the Shannon entropy of an empirical distribution of the set of feature values.

7. The method of any of previous claims, further comprising:

comparing the set of feature values with a given third threshold for each time-frequency window satisfying the first and/or second threshold, thus defining, for each time-frequency window satisfying the first and/or second threshold, a subset of the filtered set of physiological signals, called relevant filtered physiological signals; and
accumulating all relevant filtered physiological signals across time-frequency windows satisfying the first and/or second threshold, thus defining a subset of the set of physiological signals, called relevant physiological signals.

8. The method of any of previous claims, wherein the plurality of time-frequency windows overlap or do not overlap with each other and/or have an equal or a different width.

9. The method of any of previous claims, wherein the plurality of time-frequency windows are nested windows with initial bound fixed at said minimum time instant and with increasing final bound.

10. The method of any of previous claims, wherein the physiological event is an epileptic seizure or the brain response to a delivered stimulus, and wherein the physiological signals are intracranial electroencephalography, iEEG, or scalp electroencephalography, EEG, signals.

11. The method of any of previous claims, wherein the first and/or the second threshold is the same for all the time-frequency windows

12. The method of claim 1, wherein the first and second thresholds are determined using a machine learning algorithm applied on the set of physiological signals with prior information on the time-frequency windows of interest.

13. The method of claim 7, wherein the first, second and/or third thresholds are determined using a machine learning algorithm applied on the set of physiological signals with prior information on the time-frequency windows of interest and the relevant physiological signals.

14. A computer readable medium containing a computer program product for identifying time-frequency features of physiological events, the computer program product comprising program instructions that based on:

   a time period in which a physiological event occurred;
   a set of physiological signals associated with said physiological event, each signal of said set of physiological signals corresponding to a different spatial location of a body part of a living being;
   a time-frequency region of interest, said time-frequency region being defined by a minimum and a maximum time instant and a minimum and a maximum frequency, wherein said minimum and maximum time instants are comprised within said time period in which the physiological event occurred and said maximum frequency is lower or equal than the sampling rate of the physiological signals; and
   a plurality of time-frequency windows defined on the time-frequency region of interest:

      filter the set of physiological signals within each of said plurality of time-frequency windows, obtaining as a result a filtered set of physiological signals for each time-frequency window;
      calculate, for each defined time-frequency window, a given feature for the filtered set of physiological signals, each one of the signals of the filtered set of physiological signals having a given feature value, providing for each time-frequency window a set of feature values; and
      calculate, for each time-frequency window, at least one of:

         a first quantifier defined as a function of said set of features values and comparing the calculated first quantifier with a given first threshold, the program instructions further selecting the time-frequency windows satisfying the first threshold; and/or
         a second quantifier defined as a function of an empirical distribution of the set of feature values and comparing the calculated second quantifier with to a given second threshold, the program instructions further selecting the time-frequency windows satisfying the second threshold.

15. The computer readable medium of claim 14, wherein the program instructions further:

      compare the set of feature values with a given third threshold for each time-frequency window satisfying the first and/or second threshold, thus defining, for each time-frequency window satisfying the first and/or second threshold, a subset of the filtered set of physiological signals, called relevant filtered physiological signals; and
      accumulate all relevant filtered physiological signals across time-frequency windows satisfying the first and/or second threshold, thus defining a subset of the set of physiological signals, called relevant physiological signals.

**Patentansprüche**

1. Computerimplementiertes Verfahren zum Identifizieren von Zeit-Frequenz-Merkmalen von physiologischen Ereignissen, wobei das Verfahren Folgendes umfasst:

   a) Empfangen, durch ein Rechensystem:

   einer Zeitperiode, in der ein physiologisches Ereignis stattgefunden hat;
   eines Satzes von physiologischen Signalen, die mit dem physiologischen Ereignis verknüpft sind, wobei jedes Signal des Satzes von physiologischen Signalen einem anderen räumlichen Standort eines Körperteils eines Lebewesens entspricht;
   einer Zeit-Frequenz-Region von Interesse, wobei die Zeit-Frequenz-Region durch einen minimalen und einen maximalen Zeitpunkt und eine minimale und eine maximale Frequenz definiert ist, wobei der minimale und der maximale Zeitpunkt innerhalb der Zeitperiode umfasst sind, in der das physiologische Ereignis stattgefunden hat, und die maximale Frequenz niedriger oder gleich der Abtastrate von den physiologischen Signalen ist; und
   einer Vielzahl von Zeit-Frequenz-Fenstern, die auf der Zeit-Frequenz-Region von Interesse definiert sind;

   b) Filtern des Satzes von physiologischen Signalen innerhalb jedes der Vielzahl von Zeit-Frequenz-Fenstern, wobei als Ergebnis ein gefilterter Satz von physiologischen Signalen für jedes Zeit-Frequenz-Fenster erhalten wird;
   c) Berechnen eines gegebenen Merkmals für den gefilterten Satz von physiologischen Signalen für jedes definierte Zeit-Frequenz-Fenster durch das Rechensystem, wobei jedes der Signale des gefilterten Satzes von physiologischen Signalen einen gegebenen Merkmalswert aufweist, wobei für jedes Zeit-Frequenz-Fenster ein Satz von Merkmalswerten bereitgestellt wird; und
   d) Berechnen durch das Rechensystem für jedes Zeit-Frequenz-Fenster mindestens eines von:

   einem ersten Quantifizierer, der als eine Funktion des Satzes von Merkmalswerten definiert ist, und Vergleichen des berechneten ersten Quantifizierers mit einem gegebenen ersten Schwellenwert, wobei das Rechensystem ferner die Zeit-Frequenz-Fenster auswählt, die den ersten Schwellenwert erfüllen; und/oder
   einem zweiten Quantifizierer, der als eine Funktion einer empirischen Verteilung des Satzes von Merkmalswerten definiert ist, und Vergleichen des berechneten zweiten Quantifizierers mit einem gegebenen zweiten Schwellenwert, wobei das Rechensystem ferner die Zeit-Frequenz-Fenster auswählt, die den zweiten Schwellenwert erfüllen.

2. Verfahren nach Anspruch 1, wobei das gegebene Merkmal, das in Schritt c) berechnet wird, ein intrinsisches Attribut von jedem Signal des gefilterten Satzes von physiologischen Signalen definiert, wobei das intrinsische Attribut mindestens eines von der Leistung im Band (PIB) von jedem Signal innerhalb jedes Zeit-Frequenz-Fensters oder der mittleren Aktivierung (MA) beinhaltet, definiert als die momentane Aktivierung jedes Signals, gemittelt innerhalb jedes Zeit-Frequenz-Fensters, wobei die momentane Aktivierung eine kontinuierliche Leistung ist, ausgedrückt als ein z-Wert in Bezug auf eine gemeinsame präiktale Basislinienverteilung, definiert durch Zusammenfassen aller Leistungswerte der Signale innerhalb dieses Bandes.

3. Verfahren nach Anspruch 1, wobei das gegebene Merkmal in Schritt c) ein Attribut definiert, das angibt, wie jedes Signal des gefilterten Satzes von physiologischen Signalen bezüglich der anderen Signale des gefilterten Satzes in Beziehung steht, wobei das Attribut mindestens eines von der durchschnittlichen Pearson-Korrelation, der durchschnittlichen gegenseitigen Information, der Betweenness-Zentralität jedes Signals bezüglich aller anderen Signale innerhalb jedes Zeit-Frequenz-Fensters beinhaltet.

4. Verfahren nach einem der vorhergehenden Ansprüche, wobei der erste Quantifizierer mindestens eines von dem Mittelwert, der Standardabweichung, dem Maximum, einer globalen Aktivierung, GA, dem Minimum oder einer globalen Inaktivierung, GI, des Satzes von Merkmalswerten umfasst, wobei die GA als der gewichtete Durchschnitt des Satzes von positiven Merkmalswerten definiert ist, wobei jeder Merkmalswert für sich gewichtet wird, und wobei die GI als der gewichtete Durchschnitt des Satzes von negativen Merkmalswerten definiert ist, wobei jeder Merkmalswert für sich gewichtet wird.

5. Verfahren nach einem der vorhergehenden Ansprüche, wobei der zweite Quantifizierer mindestens eines von der Renyi-Entropie, den Fisher-Informationen oder einer Aktivierungsentropie, AE, einer empirischen Verteilung des Satzes von Merkmalswerten umfasst, wobei die AE als die Shannon-Entropie der empirischen Verteilung definiert ist.

6. Verfahren nach einem der vorhergehenden Ansprüche 1 bis 3, wobei der erste Quantifizierer eine globale Aktivierung, GA, umfasst, ein Maß, das als der gewichtete Durchschnitt des Satzes von positiven Merkmalswerten definiert ist, wobei jeder Merkmalswert für sich gewichtet wird, und wobei der zweite Quantifizierer eine Aktivierungsentropie, AE, umfasst, ein Maß, das als die Shannon-Entropie einer empirischen Verteilung des Satzes von Merkmalswerten definiert ist.

7. Verfahren nach einem der vorhergehenden Ansprüche, ferner Folgendes umfassend:

Vergleichen des Satzes von Merkmalswerten mit einem gegebenen dritten Schwellenwert für jedes Zeit-Frequenz-Fenster, das den ersten und/oder zweiten Schwellenwert erfüllt, wodurch für jedes Zeit-Frequenz-Fenster, das den ersten und/oder zweiten Schwellenwert erfüllt, eine Teilmenge des gefilterten Satzes von physiologischen Signalen definiert wird, die als relevante gefilterte physiologische Signale bezeichnet werden; und
Akkumulieren aller relevanten gefilterten physiologischen Signale über Zeit-Frequenz-Fenster, die den ersten und/oder zweiten Schwellenwert erfüllen, wodurch eine Teilmenge des Satzes von physiologischen Signalen definiert wird, die als relevante physiologische Signale bezeichnet werden.

8. Verfahren nach einem der vorhergehenden Ansprüche, wobei die Vielzahl von Zeit-Frequenz-Fenstern einander überlappen oder nicht überlappen und/oder eine gleiche oder eine unterschiedliche Breite aufweisen.

9. Verfahren nach einem der vorhergehenden Ansprüche, wobei die Vielzahl von Zeit-Frequenz-Fenstern verschachtelte Fenster sind, deren Anfangsgrenze auf den minimalen Zeitpunkt fixiert ist und deren Endgrenze zunimmt.

10. Verfahren nach einem der vorhergehenden Ansprüche, wobei das physiologische Ereignis ein epileptischer Anfall oder die Gehirnreaktion auf einen abgegebenen Reiz ist und wobei die physiologischen Signale Signale der Intrakranialelektroenzephalographie, iEEG, oder der Elektroenzephalographie, EEG, der Kopfhaut sind.

11. Verfahren nach einem der vorhergehenden Ansprüche, wobei der erste und/oder der zweite Schwellenwert für alle Zeit-Frequenz-Fenster gleich ist.

12. Verfahren nach Anspruch 1, wobei der erste und der zweite Schwellenwert unter Verwendung eines Maschinenlernalgorithmus bestimmt werden, der auf den Satz von physiologischen Signalen mit vorherigen Informationen über die Zeit-Frequenz-Fenster von Interesse angewendet wird.

13. Verfahren nach Anspruch 7, wobei die ersten, zweiten und/oder dritten Schwellenwerte unter Verwendung eines Maschinenlernalgorithmus bestimmt werden, der auf den Satz von physiologischen Signalen mit vorherigen Informationen über die Zeit-Frequenz-Fenster von Interesse und die relevanten physiologischen Signale angewendet wird.

14. Computerlesbares Medium, das ein Computerprogrammprodukt zum Identifizieren von Zeit-Frequenz-Merkmalen von physiologischen Ereignissen enthält, wobei das Computerprogrammprodukt Programmanweisungen umfasst, die auf Folgendem basieren:

einer Zeitperiode, in der ein physiologisches Ereignis stattgefunden hat;
einem Satz von physiologischen Signalen, die mit dem physiologischen Ereignis verknüpft sind, wobei jedes Signal des Satzes von physiologischen Signalen einem anderen räumlichen Standort eines Körperteils eines Lebewesens entspricht;
einer Zeit-Frequenz-Region von Interesse, wobei die Zeit-Frequenz-Region durch einen minimalen und einen maximalen Zeitpunkt und eine minimale und eine maximale Frequenz definiert ist, wobei der minimale und der maximale Zeitpunkt innerhalb der Zeitperiode umfasst sind, in der das physiologische Ereignis stattgefunden hat, und die maximale Frequenz niedriger oder gleich der Abtastrate von den physiologischen Signalen ist; und
einer Vielzahl von Zeit-Frequenz-Fenstern, die auf der Zeit-Frequenz-Region von Interesse definiert sind;
Filtern des Satzes von physiologischen Signalen innerhalb jedes der Vielzahl von Zeit-Frequenz-Fenstern, wobei als Ergebnis ein gefilterter Satz von physiologischen Signalen für jedes Zeit-Frequenz-Fenster erhalten wird;

**EP 3 977 478 B1**

Berechnen eines gegebenen Merkmals für den gefilterten Satz von physiologischen Signalen für jedes definierte Zeit-Frequenz-Fenster, wobei jedes der Signale des gefilterten Satzes von physiologischen Signalen einen gegebenen Merkmalswert aufweist, wobei für jedes Zeit-Frequenz-Fenster ein Satz von Merkmalswerten bereitgestellt wird; und

Berechnen für jedes Zeit-Frequenz-Fenster mindestens eines von:

einem ersten Quantifizierer, der als eine Funktion des Satzes von Merkmalswerten definiert ist, und Vergleichen des berechneten ersten Quantifizierers mit einem gegebenen ersten Schwellenwert, wobei die Programmanweisungen ferner die Zeit-Frequenz-Fenster auswählen, die den ersten Schwellenwert erfüllen; und/oder

einem zweiten Quantifizierer, der als eine Funktion einer empirischen Verteilung des Satzes von Merkmalswerten definiert ist, und Vergleichen des berechneten zweiten Quantifizierers mit einem gegebenen zweiten Schwellenwert, wobei die Programmanweisungen ferner die Zeit-Frequenz-Fenster auswählen, die den zweiten Schwellenwert erfüllen.

15. Computerlesbares Medium nach Anspruch 14, wobei die Programmanweisungen ferner Folgendes durchführen:

Vergleichen des Satzes von Merkmalswerten mit einem gegebenen dritten Schwellenwert für jedes Zeit-Frequenz-Fenster, das den ersten und/oder zweiten Schwellenwert erfüllt, wodurch für jedes Zeit-Frequenz-Fenster, das den ersten und/oder zweiten Schwellenwert erfüllt, eine Teilmenge des gefilterten Satzes von physiologischen Signalen definiert wird, die als relevante gefilterte physiologische Signale bezeichnet werden; und

Akkumulieren aller relevanten gefilterten physiologischen Signale über Zeit-Frequenz-Fenster, die den ersten und/oder zweiten Schwellenwert erfüllen, wodurch eine Teilmenge des Satzes von physiologischen Signalen definiert wird, die als relevante physiologische Signale bezeichnet werden.

**Revendications**

1. Procédé mis en oeuvre par ordinateur permettant d'identifier des caractéristiques de temps-fréquence d'événements physiologiques, le procédé comprenant :

a) recevoir, par un système informatique :

une période de temps dans laquelle s'est produit un événement physiologique ;
un ensemble de signaux physiologiques associés audit événement physiologique, chaque signal dudit ensemble de signaux physiologiques correspondant à un emplacement spatial différent d'une partie du corps d'un être vivant ;
une région de temps-fréquence d'intérêt, ladite région de temps-fréquence étant définie par un instant de temps minimum et maximum et une fréquence minimum et maximum, dans lequel lesdits instants de temps minimum et maximum sont compris dans ladite période de temps dans laquelle l'événement physiologique s'est produit et ladite fréquence maximum est inférieure ou égale au taux d'échantillonnage des signaux physiologiques ; et
une pluralité de fenêtres de temps-fréquence définies dans la région de temps-fréquence d'intérêt ;

b) filtrer l'ensemble de signaux physiologiques dans chacune de ladite pluralité de fenêtres de temps-fréquence, obtenant come résultat un ensemble filtré de signaux physiologiques pour chaque fenêtre de temps-fréquence ;
c) calculer, par le système informatique, pour chaque fenêtre de temps-fréquence définie, une caractéristique donnée pour l'ensemble filtré de signaux physiologiques, chacun des signaux de l'ensemble filtré de signaux physiologiques ayant une valeur de caractéristique donnée, fournissant pour chaque fenêtre de temps-fréquence un ensemble de valeurs de caractéristique ; et
d) calculer, par le système informatique, pour chaque fenêtre de temps-fréquence, au moins l'un de :

un premier quantificateur défini comme une fonction dudit ensemble de valeurs de caractéristique et comparer le premier quantificateur calculé à un premier seuil donné, le système informatique sélectionnant en outre les fenêtres de temps-fréquence satisfaisant le premier seuil ; et/ou
un deuxième quantificateur défini comme une fonction d'une distribution empirique dudit ensemble de valeurs de caractéristique et comparer le deuxième quantificateur calculé à un deuxième seuil donné, le

**17**

système informatique sélectionnant en outre les fenêtres de temps-fréquence satisfaisant le deuxième seuil.

2. Procédé selon la revendication 1, dans lequel ladite caractéristique donnée calculée dans l'étape c) définit un attribut intrinsèque de chaque signal de l'ensemble filtré de signaux physiologiques, l'attribut intrinsèque comportant au moins l'un de la puissance dans la bande (PIB) de chaque signal dans chaque fenêtre de temps-fréquence ou l'activation moyenne (MA), définie comme l'activation instantanée de chaque signal moyennée dans chaque fenêtre de temps-fréquence, ladite activation instantanée étant une puissance continue exprimée sous la forme d'un score z par rapport à une distribution de base précritique commune définie en regroupant toutes les valeurs de puissance des signaux dans cette bande.

3. Procédé selon la revendication 1, dans lequel ladite caractéristique donnée dans l'étape c) définit un attribut indiquant comment chaque signal de l'ensemble filtré de signaux physiologiques est lié par rapport aux autres signaux de l'ensemble filtré, ledit attribut comportant au moins l'un d'une corrélation de Pearson moyenne, l'information mutuelle moyenne, la centralité d'intermédiarité de chaque signal par rapport à tous les autres signaux dans chaque fenêtre de temps-fréquence.

4. Procédé selon l'une quelconque des revendications précédentes, dans lequel le premier quantificateur comprend au moins l'un de la moyenne, l'écart type, le maximum, une activation globale, GA, le minimum, ou une inactivation globale, GI, de l'ensemble de valeurs de caractéristique, dans lequel la GA est définie comme la moyenne pondérée de l'ensemble de valeurs de caractéristique positives, où chaque valeur de caractéristique est pondérée par elle-même, et dans lequel la GI est définie comme la moyenne pondérée de l'ensemble de valeurs de caractéristique négatives, où chaque valeur de caractéristique est pondérée par elle-même.

5. Procédé selon l'une quelconque des revendications précédentes, dans lequel le deuxième quantificateur comprend au moins l'un de l'entropie de Renyi, l'information de Fisher ou une entropie d'activation, AE, d'une distribution empirique de l'ensemble de valeurs de caractéristique, dans lequel l'AE est définie comme l'entropie de Shannon de ladite distribution empirique.

6. Procédé selon l'une quelconque des revendications précédentes 1 à 3, dans lequel le premier quantificateur comprend une activation globale, GA, une mesure définie comme la moyenne pondérée de l'ensemble de valeurs de caractéristique positives, où chaque valeur de caractéristique est pondérée par elle-même, et dans lequel le deuxième quantificateur comprend une entropie d'activation, AE, une mesure définie comme l'entropie de Shannon d'une distribution empirique de l'ensemble de valeurs de caractéristique.

7. Procédé selon l'une quelconque des revendications précédentes, comprenant en outre :

comparer l'ensemble de valeurs de caractéristique à un troisième seuil donné pour chaque fenêtre de temps-fréquence satisfaisant le premier et/ou le deuxième seuil, définissant ainsi, pour chaque fenêtre de temps-fréquence satisfaisant le premier et/ou le deuxième seuil, un sous-ensemble de l'ensemble filtré de signaux physiologiques, appelés signaux physiologiques filtrés pertinents ; et
accumuler tous les signaux physiologiques filtrés pertinents sur les fenêtres de temps-fréquence satisfaisant le premier et/ou le deuxième seuil, définissant ainsi un sous-ensemble de l'ensemble de signaux physiologiques, appelés signaux physiologiques pertinents.

8. Procédé selon l'une quelconque des revendications précédentes, dans lequel la pluralité de fenêtres de temps-fréquence se chevauchent ou ne se chevauchent pas les unes aux autres et/ou ont une largeur égale ou différente.

9. Procédé selon l'une quelconque des revendications précédentes, dans lequel la pluralité de fenêtres de temps-fréquence sont des fenêtres imbriquées avec une limite initiale fixée audit instant de temps minimum et avec une limite finale croissante.

10. Procédé selon l'une quelconque des revendications précédentes, dans lequel l'événement physiologique est une crise d'épilepsie ou la réponse cérébrale à un stimulus délivré, et dans lequel les signaux physiologiques sont des signaux d'électroencéphalographie intracrânîenne, iEEG, ou d'électroencéphalographie de cuir chevelu, EEG.

11. Procédé selon l'une quelconque des revendications précédentes, dans lequel le premier et/ou le deuxième seuil sont le même pour toutes les fenêtres de temps-fréquence

**12.** Procédé selon la revendication 1, dans lequel le premier et le deuxième seuils sont déterminés en utilisant un algorithme d'apprentissage automatique appliqué sur l'ensemble de signaux physiologiques avec des informations préalables sur les fenêtres de temps-fréquence d'intérêt.

**13.** Procédé selon la revendication 7, dans lequel le premier, le deuxième et/ou le troisième seuils sont déterminés en utilisant un algorithme d'apprentissage automatique appliqué sur l'ensemble de signaux physiologiques avec des informations préalables sur les fenêtres de temps-fréquence d'intérêt et les signaux physiologiques pertinents.

**14.** Support lisible par ordinateur contenant un produit de programme informatique permettant d'identifier des caractéristiques de temps-fréquence d'événements physiologiques, le produit de programme informatique comprenant des instructions de programme qui, sur la base de :

une période de temps dans laquelle s'est produit un événement physiologique ;
un ensemble de signaux physiologiques associés audit événement physiologique, chaque signal dudit ensemble de signaux physiologiques correspondant à un emplacement spatial différent d'une partie du corps d'un être vivant ;
une région de temps-fréquence d'intérêt, ladite région de temps-fréquence étant définie par un instant de temps minimum et maximum et une fréquence minimum et maximum, dans lequel lesdits instants de temps minimum et maximum sont compris dans ladite période de temps dans laquelle l'événement physiologique s'est produit et ladite fréquence maximum est inférieure ou égale au taux d'échantillonnage des signaux physiologiques ; et
une pluralité de fenêtres de temps-fréquence définies dans la région de temps-fréquence d'intérêt :

filtrent l'ensemble de signaux physiologiques dans chacune de ladite pluralité de fenêtres de temps-fréquence, obtenant comme résultat un ensemble filtré de signaux physiologiques pour chaque fenêtre de temps-fréquence ;
calculent, pour chaque fenêtre de temps-fréquence définie, une caractéristique donnée pour l'ensemble filtré de signaux physiologiques, chacun des signaux de l'ensemble filtré de signaux physiologiques ayant une valeur de caractéristique donnée, fournissant pour chaque fenêtre de temps-fréquence un ensemble de valeurs de caractéristique ; et
calculent, pour chaque fenêtre de temps-fréquence, au moins l'un de :

un premier quantificateur défini comme une fonction dudit ensemble de valeurs de caractéristique et comparent le premier quantificateur calculé à un premier seuil donné, les instructions de programme sélectionnent en outre les fenêtres de temps-fréquence satisfaisant le premier seuil ; et/ou
un deuxième quantificateur défini comme une fonction d'une distribution empirique de l'ensemble de valeurs de caractéristique et comparent le deuxième quantificateur calculé à un deuxième seuil donné, les instructions de programme sélectionnent en outre les fenêtres de temps-fréquence satisfaisant le deuxième seuil.

**15.** Support lisible par ordinateur selon la revendication 14, dans lequel, en outre, les instructions programme :

comparent l'ensemble de valeurs de caractéristique à un troisième seuil donné pour chaque fenêtre de temps-fréquence satisfaisant le premier et/ou deuxième seuil, définissant ainsi, pour chaque fenêtre de temps-fréquence satisfaisant le premier et/ou le deuxième seuil, un sous-ensemble de l'ensemble filtré de signaux physiologiques, appelés signaux physiologiques filtrés pertinents ; et
accumulent tous les signaux physiologiques filtrés pertinents sur les fenêtres de temps-fréquence satisfaisant le premier et/ou le deuxième seuil, définissant ainsi un sous-ensemble de l'ensemble de signaux physiologiques, appelés signaux physiologiques pertinents.

```
                    ┌──────────┐
                    │  Start   │ ⌇ 1001
                    └────┬─────┘
                         ▼
┌─────────────────────────────────────────────────┐
│ Filter a set of physiological signals within each │ ⌇ 1002
│ of a plurality of time-frequency windows,          │
│ providing a filtered set                           │
└─────────────────────┬───────────────────────────┘
                      ▼
┌─────────────────────────────────────────────────┐
│ Calculate, for each time-frequency window, a      │ ⌇ 1003
│ given feature for the filtered set                 │
└─────────────────────┬───────────────────────────┘
          ┌───────────┴────────────┐
          ▼                         ▼
┌────────────────────────┐ ┌────────────────────────┐
│ Calculate, for each time-│ │ Calculate, for each time-│ ⌇ 1007
│ frequency window, a first │ │ frequency window, a second│
│ quantifier defined as a   │ │ quantifier defined as a   │
1004 │ function of a set of │ │ function of an empirical  │
│ feature values           │ │ distribution of a set of  │
│                          │ │ feature values            │
└───────────┬────────────┘ └───────────┬────────────┘
            ▼                           ▼
┌────────────────────────┐ ┌────────────────────────┐
1005 │ Compare the first │ │ Compare the second │ ⌇ 1008
│ quantifier with a threshold│ │ quantifier with a threshold│
└───────────┬────────────┘ └───────────┬────────────┘
            ▼                           ▼
┌────────────────────────┐ ┌────────────────────────┐
1006 │ Select the time- │ │ Select the time- │ ⌇ 1009
│ frequency windows        │ │ frequency windows        │
│ satisfying the threshold  │ │ satisfying the threshold  │
└────────────────────────┘ └────────────────────────┘
```

**Fig. 1**

20

**Fig. 2A**

**Fig. 2B**

# Fig. 2C

(A) 12-31 Hz, 0-20 s

(B) 107-130 Hz, 0-100 ms

(D) 31-50 Hz, 0-5 s

(C) 107-130 Hz, 0-10 s

EP 3 977 478 B1

**Fig. 3A**

**Fig. 3B**

Activation entropy (AE)

Length of time window
after seizure onset (s)

# Fig. 3C

# Fig. 4

**(A)** Signals are band-pass filtered in different bands of interest

**(B)** MA is obtained over different time windows of interest

**(C)** For each frequency and time window of interest, AE and GA are obtained

**(D)** SOW selection is achieved by maximizing GA while keeping AE small

**(E)** For each SOW, regions with the highest MAs are selected

**(F)** The seizure onset zone (SOZ) is delineated by accumulating all selected regions across SOWs

Fig. 5A

Fig. 5B

Fig. 5C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9326698 B2
- US 6678548 B1
- US 2012245481 A1
- US 2016045127 A1
- US 2016287118 A1
- WO 2018005981 A1
- US 6594524 B2

### Non-patent literature cited in the description

- Detection of recurrent activation patterns across focal seizures: Application to seizure onset zone identification. **VILA-VIDALET**. CLINICAL NEUROPHYSIOLOGY. ELSEVIER, 03 April 2017, vol. 128, 977-985
- **ANDRZEJAK RG** ; **DAVID O** ; **GNATKOVSKY V** ; **WENDLING F** ; **BARTOLOMEI F** ; **FRANCIONE S et al.** Localization of epileptogenic zone on pre-surgical intracranial EEG recordings: toward a validation of quantitative signal analysis approaches. *Brain Topography*, 2015, vol. 28 (6), 832-837
- **BARTOLOMEI F** ; **CHAUVEL P** ; **WENDLING F**. Epileptogenicity of brain structures in human temporal lobe epilepsy: a quantified study from intracerebral EEG. *Brain*, 2008, vol. 131 (7), 1818-1830
- **DAVID O** ; **BLAUWBLOMME T** ; **JOB AS** ; **CHABARDÈS S** ; **HOFFMANN D** ; **MINOTTI L et al.** Imaging the seizure onset zone with stereo-electroencephalography. *Brain*, 2011, vol. 134 (10), 2898-2911
- **GEERTSEMA EE** ; **VISSER GH** ; **VELIS DN** ; **CLAUS SP** ; **ZIJLMANS M** ; **KALITZIN SN**. Automated seizure onset zone approximation based on nonharmonic high-frequency oscillations in human interictal intracranial eegs. *International Journal of Neural Systems*, 2015, vol. 25 (05), 1550015
- **GENER T** ; **TAUSTE CAMPO A** ; **ALEMANY-GONZÁLEZ M** ; **NEBOT P** ; **DELGADO-SALIENT C** ; **CHANOVAS J**. Puig MV. Serotonin 5-HT1A, 5-HT2A and dopamine D2 receptors strongly influence prefronto-hippocampal neural networks in alert mice. *Contribution to the actions of risperidone Neuropharmacology*, 2019, vol. 158, 107743, https://doi.org/10.1016/j.neuropharm.2019.107743
- **GNATKOVSKY V** ; **FRANCIONE S** ; **CARDINALE F** ; **MAI R** ; **TASSI L** ; **LO RUSSO G et al.** Identification of reproducible ictal patterns based on quantified frequency analysis of intracranial EEG signals. *Epilepsia*, 2011, vol. 52 (3), 477-488
- **GNATKOVSKY V** ; **CURTIS M** ; **PASTORI C** ; **CARDINALE F** ; **LO RUSSO G** ; **MAI R et al.** Biomarkers of epileptogenic zone defined by quantified stereo-EEG analysis. *Epilepsia*, 2014, vol. 55 (2), 296-305
- **LIU S** ; **SHA Z** ; **SENCER A** ; **AYDOSELI A** ; **BEBEK N** ; **ABOSCH A et al.** Exploring the time-frequency content of high frequency oscillations for automated identification of seizure onset zone in epilepsy. *Journal of Neural Engineering*, 2016, vol. 13 (2), 026026
- A novel HFO-based method for unsupervised localization of the seizure onset zone in drug-resistant epilepsy. In. **MURPHY PM** ; **PATERNOS AJ** ; **SANTANIELLO S**. Engineering in Medicine and Biology Society (EMBC). IEEE, 2017, 1054-1057
- Inter-ictal seizure onset zone localization using unsupervised clustering and bayesian filtering. **VARATHARAJAH Y** ; **BERRY BM** ; **KALBARCZYK ZT** ; **BRINKMANN BH** ; **WORRELL GA** ; **IYER RK**. Neural Engineering (NER. IEEE, 2017, 533-539
- **VILA-VIDAL M** ; **PRINCIPE A** ; **LEY M** ; **DECO G** ; **TAUSTE CAMPO A** ; **ROCAMORA R**. Detection of recurrent activation patterns across focal seizures: Application to seizure onset zone identification. *Clinical Neurophysiology*, 2017, vol. 128 (6), 977-985